# EUROPÄISCHE PATENTANMELDUNG

(11) **EP 3 315 127 A1**
(43) Veröffentlichungstag der Anmeldung: **02.05.2018**
(21) Anmeldenummer: 17001773.5
(22) Anmeldetag: 26.10.2017
(51) Int. Cl.: A61K 31/14, A61K 31/197, A61K 31/4415, A61K 33/06, A61K 36/16, A61K 36/258

(54) **STOFFMISCHUNG ZUR VERBESSERUNG DER MENTALEN UND KOGNITIVEN PROZESSE**

(30) Priorität: 31.10.2016 DE 202016006697 U
(71) Anmelder: Leopold, Kati, 14478 Potsdam (DE)
(72) Erfinder: Leopold, Kati, 14478 Potsdam (DE)
(74) Vertreter: Hertin und Partner Rechts- und Patentanwälte PartG mbB

(57) **Zusammenfassung**

Stoffmischung zur Verbesserung der mentalen und kognitiven Prozesse, umfassend, DMAE in einer Menge von 20 mg bis 180 mg, vorzugsweise in einer Menge von 40 mg bis 110 mg, besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, dadurch gekennzeichnet, dass die Stoffmischung Ginkgo in einer Menge von 20 bis 240 mg, vorzugsweise in einer Menge von 40 mg bis 120 mg, besonders bevorzugt in einer Menge von 40 bis 60 mg, umfasst.

## Beschreibung

Die Erfindung bezieht sich auf eine Stoffmischung gemäß dem Oberbegriff des Anspruchs 1. Durch die vorliegende Erfindung werden die mentalen und kognitiven Prozesse verbessert, insbesondere die Konzentrationsfähigkeit und geistige Leistungsfähigkeit.

Präparate, die den Stoff DMAE (Dimethylaminoethanol) enthalten bzw. auf diesem basieren, sind als Arzneimittel und Nahrungsergänzungsmittel bekannt. Nach wie vor bestehen bei den handelsüblichen Präparaten verschiedene Nachteile, so dass Bedarf an Alternativpräparaten bzw. an Präparaten mit verbesserten Eigenschaften besteht. Gerade im Hinblick auf die pharmakologische Wirksamkeit, die Bioverfügbarkeit/Wirkstofffreisetzung/Blutspiegelwerte, die Nebenwirkungen und die Schnelligkeit des Wirkeintritts besteht Optimierungsbedarf. Zudem ist bei Anwendung von DMAE-haltigen Produkten ein Gewöhnungseffekt bekannt, der zu Dosiserhöhungen führt. Auch von ähnlich wirkenden, chemisch verwandten Stoffen wie Procainhydrochlorid sind Gewöhnungseffekte und Suchtpotential bekannt.

Neben eines teilweise zu schwachen pharmakologischen Effektes bei niedrigen Konzentrationen bei breiten Anwendungsgruppen/Patientengruppen zeigte sich auch, dass die Wirkung bei Teilen der Anwender bzw. Patienten bei mittlerer und hoher Dosierung so schwach ausfiel, dass die Dosis wesentlich erhöht werden musste. Die Dosiserhöhungen waren dann mit verstärktem Auftreten von Nebenwirkungen verbunden. Bei DMAE-haltigen Präparaten bestand somit die Notwendigkeit, neue optimierte Formulierungen mit verbesserten Eigenschaften zu finden.

Die vorliegende Erfindung gemäß Anspruch 1 schafft hier Abhilfe. Der Erfindung liegt die Aufgabe zugrunde, eine optimierte DMAE-haltige Stoffmischung bereitzustellen. Gelöst wurde diese Aufgabe durch eine Stoffmischung, welche die Merkmale des Anspruchs 1 aufweist.

Die Stoffmischung der vorliegenden Erfindung umfasst eine einzigartige, innovative Kombination aus neuroprotektiven Stoffen, die gezielt aufeinander qualitativ und quantitativ abgestimmt sind. Überraschenderweise zeigte sich, dass die Stoffmischung gemäß Anspruch 1 wesentliche synergistische Vorteile der einzelnen Stoffkomponenten zueinander aufweist. Die durch die Erfindung erreichten Vorteile sind im Wesentlichen darin zu sehen, dass dank der erfindungsgemäßen Stoffmischung der pharmakologische Effekt bei einem hohen Anteil der Anwender/Patienten sehr ausgeprägt ist, wobei die eingesetzte DMAE-Menge meist im mittleren oder niedrigen Bereich ist (unter 200 mg DMAE). Der Anteil der Anwender/Patienten, die einen zu schwachen pharmakologischen Effekt bei niedrigen und mittleren Mengen verspüren, konnte reduziert werden, so dass bei diesen Anwendern/Patienten auf hohe Dosierungen verzichtet werden konnte. Durch die Vermeidung von hohen Dosen (über 200 mg DMAE) kann somit die Nebenwirkungsrate reduziert werden. Ferner hat sich gezeigt, dass sich auch niedrigen und mittleren Dosierungen das generelle Nebenwirkungsprofil verbessert hat. Ein weiterer Vorteil besteht darin, dass bei jungen Menschen der pharmakologische Effekt besonders vorteilhaft eintritt. Bei Anwendung von DMAE-haltigen Produkten ist bekannt, dass relativ schnell ein Gewöhnungseffekt auftreten konnte, wenn DMAE zur Steigerung der Konzentration eingesetzt wurde. Dieser Gewöhnungseffekt tritt weniger oft und später ein, wenn die vorliegende Erfindung verwendet wird, um die Konzentrationsfähigkeit kurz- und mittelfristig zu verbessern.

Nach diversen Versuchsreihen haben sich bevorzugte Ausführungsvarianten der Erfindung herausgestellt, die nachfolgend offenbart werden. Der Ausdruck "mentale und kognitive Prozesse", der nachfolgendend verwendet wird, bezieht sich u.a. aber nicht ausschließlich auf die Konzentrationsfähigkeit, das Erinnerungsvermögen, die geistige Vitalität, die Stimmung und die allgemeine geistige Leistungsfähigkeit.

Dimethylaminoethanol (DMAE) ist ein Precursor des Neurotransmitters Acetylcholin im menschlichen Hirn und der neuronalen Membrankomponente Phosphatidylcholin. DMAE beschleunigt die Synthese von Phosphatidylcholin und die Einlagerung von Phosphatidylcholin in die neuronalen Zellmembranen. Innerhalb des Hirns hat DMAE antioxidative Eigenschaften und stimuliert den oxidativen Metabolismus in der Großhirnrinde. Studien zufolge kann durch DMAE die Lebenszeit von Zellen um 50 Prozent verlängert werden. DMAE findet Anwendung zur kurzfristigen Verbesserung der Konzentrationsfähigkeit und Aufmerksamkeit als eine Art Stimulanzmittel, aber auch als Nootropikum und Antidementivum. DMAE überwindet die Gehirnschranke schneller als Cholin. DMAE fördert Gedächtnis- und Lernleistungen und kann auch bei hyperaktiven Kindern oder solchen mit Konzentrationsstörungen eingesetzt werden.

Präparate auf DMAE-Basis werden oft bei Depressionen, chronischer Müdigkeit und Antriebsarmut eingesetzt. Positive Ergebnisse wurden auch bei seniler Dementia, Phobien und Schizophrenien erzielt. Die erfindungsgemäße Stoffmischung kann daher auch bei den vorgenannten Indikationen eingesetzt werden. DMAE entfaltet kurz- und langfristige Wirkungen, wobei letztere nach einigen Monaten eintreten. Sowohl alte Anwender/Patienten als auch junge Anwender/Patienten profitieren von der Verbesserung der mentalen und kognitiven Prozesse von DMAE.

Vorzugsweise umfasst die erfindungsgemäße Stoffmischung DMAE in einer Menge von 20 bis 180 mg. In speziellen Ausführung umfasst die erfindungsgemäße Stoffmischung DMAE in Mengen von 40 mg bis 110 mg. In besonders bevorzugten Ausführungsformen umfasst die erfindungsgemäße Stoffmischung DMAE in einer Menge von 45 mg bis 55 mg, wobei sich die gemachten Mengenangaben von DMAE auf das freie Amin ohne Gegenion beziehen (Molare Masse: 89,14 g/mol). In Ergänzung zu DMAE oder alternativ umfassen besondere Ausführungen der Erfindung die Derivate von DMAE, die alle in den Ernährungswissenschaften und in der Pharmazie bekannten Salze wie Hydrochloride oder sonstige Verbindungen von DMAE wie Tartrate umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates relativ entsprechend), so dass die gleichen Mengen an DMAE beim Derivat bei den bevorzugten Ausführungsformen vorliegen. Alternative Bezeichnungen für DMAE sind neben Dimethylaminoethanol 2-(dimethylamino)ethanol, beta-dimethyl-aminoethyl-Alkohol und N,N-dimethyl-2-hydroxyethlylamin.

Cholin spielt eine zentrale Rolle im Hirnstoffwechsel. Durch die Übertragung eines Acetyl-Restes auf das Cholin durch die Cholin-Acetyltransferase erfolgt die Biosynthese des wichtigen Neurotransmitters Acetylcholin in den sogenannten cholinergen Fasern. Bei Bedarf, z. B. bei einer konzentrierten Tätigkeit, wird das gespeicherte Cholin in Acetylcholin umgewandelt. Die erfindungsgemäße Stoffmischung umfasst Cholin, vorzugsweise in einer Menge von 25 mg bis 190 mg, bei besonders bevorzugten Varianten in einer Menge 40 mg bis 120 mg. Spezielle Varianten der Erfindung umfassen Cholin in einer Menge von 45 bis 55 mg, wobei sich die gemachten Mengenangaben von Cholin auf das freie Amin beziehen (Molare Masse: 104,171 g/mol). In Ergänzung zu Cholin oder alternativ umfassen besondere Ausführungen der Erfindung die Derivate von Cholin, die alle in den Ernährungswissenschaften und in der Pharmazie bekannten Salze von Cholin wie Hydrochloride oder sonstige Verbindung wie Hydroxide umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivats entsprechend relativ), so dass die gleichen Mengen an Cholin beim Derivat bei den bevorzugten Ausführungsformen vorliegen.

Ginkgo verbessert die Gedächtnisleistung, Merkfähigkeit, Konzentration und allgemeine mentale Leistung, was durch verschiedene Studien belegt werden konnte. Die Durchblutung des Hirns wird gefördert, und die Hirnleistung dadurch erhöht. Die Wirkung des Ginkgos basiert auf einer Verbesserung der Kommunikation der Zellen des Gehirns. Die vorliegende Erfindung umfasst Ginkgo, vorzugsweise Ginkgo-Extrakt (Ginkgo-Extrakt wird nachfolgend nur als Ginkgo bezeichnet), wobei vorzugsweise der eingesetzte Ginkgo den Vorgaben des Europäischen Arzneibuchs und des Deutschen Arzneibuchs entspricht, vorzugsweise wird daher der gereinigte und quantifizierte Ginkgotrockenextrakt aus den getrockneten Blättern von Ginkgo biloba L. hergestellt, wobei der Trockenextrakt vorzugsweise folgende Zusammensetzung umfasst: 22 bis 27 Prozent Flavonoide als Flavonglykoside mit einer mittleren Molmasse von 756,7 g/mol sowie 5 bis 7 Prozent Terpenlaktone, davon 2,8 bis 3,4 Prozent Ginkgolide A, B und C sowie 2,6 bis 3,2 Prozent Bilobalid und weniger als 5 ppm Ginkgosäuren. Die vorliegende Erfindung umfasst vorzugsweise Ginkgo, besonders bevorzugt in einer Menge von 20 bis 240 mg. Spezielle Ausführungen der Erfindung umfassen Ginkgo in Mengen von 40 mg bis 120 mg, wiederum besonders bevorzugte Ausführungen in Mengen von 40 mg bis 60 mg.

Vorzugsweise umfasst die vorliegende Erfindung ein Ginseng-Extrakt. Ginseng ist ein Stärkungs- und Kräftigungsmittel bei Müdigkeits- und Schwächegefühlen sowie bei nachlassender Leistungs- und Konzentrationsfähigkeit. Wichtigster Bestandteil des Ginsengs sind für die vorliegende Erfindung Ginsenoside, insbesondere das Ginsensosid Rg1, das den Blutdruck erhöht und den Kreislauf stimuliert.

Vorzugsweise wird ein Trockenextrakt aus Panax Ginseng verwendet, das den Vorgaben des Europäischen Arzneibuchs entspricht. Besonders bevorzugte Ausführungsformen des Ginsengtrochenextraktes umfassen mindestens 6% Ginsenoside (bezogen auf Rg1-Ginsenoside). Ginseng ist nachfolgend gleichbedeutend mit Ginseng-Extrakt.

Die vorliegende Erfindung umfasst vorzugsweise Ginseng, besonders bevorzugt in Mengen von 30 mg bis 180 mg. Spezielle Ausführungen umfassen Mengen an Ginseng von 40 mg bis 105 mg, besonders bevorzugt sind Mengen von 40 bis 60 mg.

Pantothensäure, auch Vitamin B5, ist ein Schlüsselbestandteil von neuroprotektiven Stoffmischungen. Pantothensäure wird in Pantethin umgewandelt, das für die Synthese von Coenzym A (CoA) wichtig ist. CoA verbessert die Hirnfunktion durch Förderung der Acetylcholinsynthese. Zudem verbessert die Pantothensäure die Aufnahme von Acetylcholin ins Hirn, was einen synergistischen Effekt zu den erfindungsgemäßen Stoffkomponenten DMAE und Cholin erklärt. Die vorliegende Erfindung umfasst vorzugsweise Pantothensäure. In bevorzugten Ausführungen umfasst die vorliegende Erfindung Pantothensäure in einer Menge von 1 mg bis 10 mg. In besonderen Ausführungen liegt Pantothensäure in einer Menge von 2 mg bis 7 mg vor. Bei besonders bevorzugten Ausführungsformen liegt Pantothensäure in einer Menge von 2,5 mg bis 3,5 mg in der erfindungsgemäßen Stoffmischung vor, wobei sich die gemachten Mengenangaben von Pantothensäure auf das freie Amin ohne Gegenion beziehen (Molare Masse: 219,23 g/mol). In Ergänzung zu Pantothensäure oder alternativ umfassen besondere Ausführungen der Erfindung die Derivate von Pantothensäure, die alle in den Ernährungswissenschaften und in der Pharmazie bekannten Salze von Pantothensäure wie Hydrochloride oder sonstige Verbindungen wie Hydroxide umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates relativ entsprechend), so dass die gleichen Mengen an Pantothensäuren beim Derivat bei den bevorzugten Ausführungsformen vorliegen.

Magnesium spielt im Nervensystem und in der Psyche eine zentrale Rolle. Es dient der Verringerung der Müdigkeit und kann Muskelkrämpfe verhindern und lindern. Bevorzugte Ausführungsformen der Erfindung umfassen Magnesium. Besonders bevorzugt sind Mengen an Magnesium von 100 mg bis 400 mg. Spezielle Ausführungen umfassen Mengen von 120 mg bis 300 mg, besondere Ausführungen umfassen 140 mg bis 160 mg. Die Mengenangaben in mg beziehen sich auf das reine Mg (molare Masse: 23,305 g/mol). In Ergänzung zu Magnesium oder alternativ umfassen besondere Ausführungen der Erfindung die Derivate von Magnesium, die alle in den Ernährungswissenschaften und in der Pharmazie bekannten Salze oder sonstige Verbindungen wie Hydroxide oder Oxide oder Magnesiumionen umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates entsprechend relativ), so dass die gleichen Mengen an Magnesium beim Derivat bei den bevorzugten Ausführungsformen vorliegen.

Alternative Ausführungen der erfindungsgemäßen Stoffmischung umfassen Thiamin (Vitamin B1). Thiamin spielt eine zentrale Rolle im Hirnstoffwechsel. Es wird auch als Stimmungsvitamin bezeichnet und unterstützt die stimmungsaufhellende Wirkung von DMAE. Die Erfindung umfasst vorzugsweise Menge von 0,3 mg bis 1,4 mg, bei besonders bevorzugten Ausführungen der Erfindung Mengen von 0,4 mg bis 1,3 mg, bei speziellen Ausführungen von 0,6 mg bis 0,66 mg. Die Mengenangaben in mg beziehen sich auf das reine Thiamin (molare Masse: 265,35 g/mol). In Ergänzung zu Thiamin oder alternativ umfassen besondere Ausführungen der Erfindung die Derivate von Thiamin, die alle in den Ernährungswissenschaften und in der Pharmazie bekannten Salze wie Hydrochloride von Thiamin oder sonstige Verbindungen wie Hydroxide umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates relativ entsprechend), so dass die gleichen Mengen an Thiamin beim Derivat bei den bevorzugten Ausführungsformen vorliegen.

Riboflavin (Vitamin B2) übernimmt verschiedene zentrale Rollen im Zellstoffwechsel. Besondere Ausführungen der Erfindung umfassen Riboflavin, vorzugsweise in einer Menge von 0,1 mg bis 1,6 mg. Spezielle Ausführungen umfassen Riboflavin in einer Menge von 0,72 bis 0,78 mg. Die Mengenangaben in mg beziehen sich auf das reine Riboflavin (molare Masse: 376,36 g/mol). In- Ergänzung zu Riboflavin oder alternativ umfassen besondere Ausführungen der Erfindung die Derivate von Riboflavin, die alle in den Ernährungswissenschaften und in der Pharmazie bekannten Salze von Riboflavin wie Hydrochloride oder sonstige Verbindungen wie Hydroxide umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates relativ entsprechend), so dass die gleichen Mengen an Riboflavin beim Derivat bei den bevorzugten Ausführungsformen vorliegen.

Pyridoxin (Vitamin B6) regelt verschiedene zentrale Abläufe im Zellstoffwechsel, ist am Aufbau und Schutz von Nervenverbindungen beteiligt und unterstützt das Immun- und Hormonsystem. Alternative Ausführungen der Erfindung umfassen Pyridoxin (Vitamin B6), vorzugsweise in einer Menge von 0,2 mg bis 2,1 mg. Besondere bevorzugte Ausführungen umfassen Pyridoxin in einer Menge von 0,9 mg bis 1,1 mg. Die Mengenangaben in mg beziehen sich auf das reine Pyridoxin (molare Masse: 169,18 g/mol). In Ergänzung zu Pyridoxin oder alternativ umfassen besondere Ausführungen der Erfindung die Derivate von Pyridoxin, die alle in den Ernährungswissenschaften und in der Pharmazie bekannten Salzevon Pyridoxin wie Hydrochloride oder sonstige Verbindungen wie Hydroxide umfassen. Bei Vorliegen von Derivatverbindungen in besonderen Ausführungen der Stoffmischung der vorliegenden Erfindung sind die Mengenangaben in mg der größeren molaren Masse des Derivates entsprechend größer (die Mengenangaben steigern sich der molaren Masse des Derivates relativ entsprechend), so dass die gleichen Mengen an Pyridoxin beim Derivat bei den bevorzugten Ausführungsformen vorliegen.

Die vorgenannten Komponenten können beliebig miteinander kombiniert werden, wobei die Merkmale des Oberbegriffs des Anspruchs 1 in den Stoffmischungen jeweils umfasst sind.

Durch die einzigartige Kombination der einzelnen Komponenten zu der erfindungsgemäßen Stoffmischung kann der Gewöhnungseffekt an DMAE, wenn es als konzentrations- und aufmerksamkeitssteigerndes Mittel eingesetzt wird, wesentlich verringert werden, insbesondere bei Anwendern unter 35 Jahren ist diese überraschende Effekt stark ausgeprägt. Zudem tritt der Wirkeffekt intensiver und schneller ein. Zu erklären ist die Wirkoptimierung mit der einzigartigen Stoffmischung sowie der optimierten Abstimmung der eingesetzten Mengen aufeinander. Ginkgo wirkt pharmakologisch im Vergleich zu DMAE und Cholin über andere Mechanismen. Es hat sich gezeigt, dass die Kombination der drei Stoffkomponenten in den Mengen und Mengenverhältnissen gemäß Anspruch 1 den Gewöhnungseffekt bei DMEAhaltigen Präparaten verzögert. Zudem sprechen mehr Anwender/Patienten überhaupt auf die Stoffmischung der Erfindung an. Der Anteil der Anwender/Patienten, die auf DMAE-haltige Präparate nicht ansprachen oder nur in sehr schwacher Weise, konnte durch die Erfindung reduziert werden. Es zeigte sich, dass dieser Anteil der Anwender/Patienten noch einmal durch Varianten der Erfindung, umfassend neben DMAE, Cholin und Gingko zusätzlich Ginseng und/oder Pantothensäure und/oder Magnesiumoxid und/oder Thiamin und/oder Riboflavin und/oder Pyridoxin, reduziert werden konnte. Ein überraschender Effekt der erfindungsgemäßen Stoffmischung besteht in der niedrigeren Nebenwirkungsrate. Während bei Präparaten mit hohen Dosen an DMAE (200 mg - 800 mg) relativ oft Fälle von Kopfschmerzen, Gereiztheit, allergische Reaktion und Muskelverspannnungen, ist das Nebenwirkungsprofil bei Verwendung der erfindungsgemäßen Stoffmischung für den Anwender/Patienten in qualitativer und quantitativer Hinsicht günstiger.

Die erfindungsgemäße Stoffmischung kann sowohl in fester als auch flüssiger Form vorliegen. Vorzugsweise wird die Stoffmischung in Form einer Kapsel verabreicht. Die erfindungsgemäße Stoffmischung kann auch in Form von Tabletten oder Dragees vorliegen.

Die nachfolgenden Mengenangaben der einzelnen Stoffkomponenten der Erfindung (u.a. DMAE, Cholin, Ginkgo (nachfolgend als Ginkgo-(troken)extrakt zu verstehen), Ginseng (nachfolgend als Ginsen-(trocken)extrakt), Pantothensäure, Magnesium, Thiamin, Riboflavin, Pyridoxin) zu besonderen Ausführungen der Erfindung können in beliebiger Weise miteinander kombiniert werden:
Vorzugsweise wird erfindungsgemäß eine Stoffmischung zur Verbesserung der mentalen und kognitiven Prozesse beansprucht, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, wobei die Stoffmischung ferner umfasst, Ginkgo in einer Menge von 20 bis 240 mg, vorzugsweise in einer Menge von 40 mg bis 120 mg, besonders bevorzugt in einer Menge von 40 bis 60 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo, vorzugsweise in einer Menge von 20 bis 240 mg, besonders bevorzugt in einer Menge von 40 mg bis 120 mg, in speziellen Ausführungen in einer Menge von 40 bis 60 mg, und wobei die beanspruchte Stoffmischung ferner Ginseng umfasst, vorzugsweise in einer Menge von 30 mg bis 180 mg, besonders bevorzugt in einer Menge von 40 mg bis 105 mg, in speziellen Ausführungen in einer Menge von 40 bis 60 mg.

Eine alternative Ausführungsform der Erfindung umfasst eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, wobei die Stoffmischung zusätzlich Pantothensäure umfasst, vorzugsweise in einer Menge von 1 mg bis 10 mg, besonders bevorzugt in einer Menge von 2 mg bis 7 mg, in speziellen Ausführungen in einer Menge von 2,5 mg bis 3,5 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, wobei die Stoffmischung in dieser Ausführungsform - Magnesium umfasst, vorzugsweise in einer Menge von 100 bis 400 mg, besonders bevorzugt in einer Menge von 120 mg bis 300 mg, in speziellen Ausführungen 140 bis 160 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, wobei die Stoffmischung zusätzlich Thiamin umfasst, vorzugsweise in einer Menge von 0,4 bis 1,7 mg, besonders bevorzugt in einer Menge von 0,5 mg bis 1,3 mg, in speziellen Ausführungen in einer Menge von 0,64 mg bis 0,66 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, wobei die Stoffmischung zusätzlich Riboflavin umfasst, vorzugsweise in einer Menge von 0,3 bis 2,0 mg, vorzugsweise in einer Menge von 0,4 mg bis 1,5 mg, besonders bevorzugt in einer Menge von 0,74 mg bis 0,76 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, optional eine der vorgenanten Riboflavin-Mengen umfassend, wobei die Stoffmischung zusätzlich Pyridoxin umfasst, vorzugsweise in Menge von 0,4 mg bis 2,8 mg, vorzugsweise in einer Menge von 0,8 mg bis 2,3 mg, besonders bevorzugt in einer Menge von 0,9 mg bis 1,1 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, optional eine der vorgenanten Riboflavin-Mengen umfassend, wobei die Stoffmischung zusätzlich Pyridoxin umfasst, vorzugsweise in Menge von 0,4 mg bis 2,8 mg, vorzugsweise in einer Menge von 0,8 mg bis 2,3 mg, besonders bevorzugt in einer Menge von 0,9 mg bis 1,1 mg.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, optional eine der vorgenanten Riboflavin-Mengen umfassend, optional eine der vorgenannten Pyridoxin-Mengen umfassend, wobei die Stoffmischung ein Nahrungsergänzungsmittel ist.

Eine alternative Ausführungsform der Erfindung ist eine Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginkgo in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, optional eine der vorgenanten Riboflavin-Mengen umfassend, optional eine der vorgenannten Pyridoxin-Mengen umfassend, wobei die erfindungsgemäße Stoffmischung ein Arzneimittel zur Verbesserung der Konzentrationsfähigkeit ist.

Verwendung einer Stoffmischung zur Verbesserung der mentalen und kognitiven Prozesse, die DMAE in einer Menge von 20 mg bis 180 mg, vorzugsweise in einer Menge von 40 mg bis 110 mg und besonders bevorzugt in einer Menge von 45 bis 55 mg, Cholin in einer Menge von 25 mg bis 190 mg, vorzugsweise in einer Menge 40 mg bis 120 mg, vorzugsweise in einer Menge von 45 bis 55 mg, umfasst, ferner umfassend Ginko in einer Menge von 20 bis 240 mg, vorzugsweise in einer Menge von 40 mg bis 120 mg, besonders bevorzugt in einer Menge von 40 bis 60 mg, optional eine der vorgenannten Ginseng-Mengen umfassend, optional eine der vorgenannten Pantothensäure-Mengen umfassend, optional eine der vorgenannten Magnesium-Mengen umfassend, optional eine der vorgenannten Thiamin-Mengen umfassend, optional eine der vorgenanten Riboflavin-Mengen umfassend, optional eine der vorgenannten Pyridoxin-Mengen umfassend, als Nahrungsergänzungsmittel, wobei bei alternativen Verwendungen die Stoffmischung zweimal täglich oder alternativ dreimal täglich eingenommen wird.

In besonderen Ausführungsformen wird die vorliegende Erfindung folgendermaßen verwendet:
Erzeugnis gemäß Anspruch 1 oder einer der oben beschriebenen Ausführungsformen zur Verwendung als Medikament gegen eines der oben genannten Leiden (z.B. Demenz, Depression, chronische Müdigkeit, Antriebsarmut oder Hyperaktivität). Erzeugnis gemäß Anspruch 1 oder einer der beschriebenen Ausführungsformen zur Verwendung bei der Behandlung eines der oben genannten Leidens (z.B. Demenz, Depression, chronische Müdigkeit, Antriebsarmut oder Hyperaktivität).

### Beispiel 1:

DMAE: 50 mg
Cholin: 50 mg
Ginkgo-Extrakt: 50 mg
Ginseng-Extrakt: 50 mg
Riboflavin: 0,75 mg
Thiamin: 0,65 mg
Pantothensäure: 3,00 mg
Pyridoxin: 1,00 mg
Magnesium: 150 mg

### Beispiel 2:

DMAE-Hydroclorid: 90 mg
Cholin-Tartrat: 60 mg
Ginkgo-Extrakt: 60 mg
Ginseng-Extrakt: 60 mg
Riboflavin: 0,75 mg
Thiamin: 0,65 mg
Pantothensäure: 3,00 mg
Pyridoxinhydrchlorid: 1,00 mg
Magnesiumoxid: 160 mg

Obige Stoffmischungen in den Beispielen wird vorzugsweise in einer Kapsel dem Anwender/Patienten bereitgestellt.

## Patentansprüche

1. Stoffmischung zur Verbesserung der mentalen und kognitiven Prozesse, umfassend, DMAE, Cholin, Ginseng, **dadurch gekennzeichnet**, dassdie Stoffmischung Ginkgo umfasst.

2. Stoffmischung gemäß Anspruch 1, **dadurch gekennzeichnet, dass** die Stoffmischung Ginkgo in einer Menge von 40 bis 120 mg umfasst.

3. Stoffmischung gemäß Anspruch 1, **dadurch gekennzeichnet, dass** die Stoffmischung Ginkgo in einer Menge von 40 bis 60 mg umfasst.

4. Stoffmischung gemäß einem der Ansprüche 1 bis 3, **dadurch gekennzeichnet, dass** die Stoffmischung DMAE in einer Menge von 40 bis 110 mg umfasst.

5. Stoffmischung gemäß einem der Ansprüche 1 bis 3, **dadurch gekennzeichnet, dass** die Stoffmischung DMAE in einer Menge von 45 bis 55 mg umfasst.

6. Stoffmischung gemäß einem der Ansprüche 1 bis 5, **dadurch gekennzeichnet, dass** die Stoffmischung Cholin in einer Menge von 40 bis 120 mg umfasst.

7. Stoffmischung gemäß einem der Ansprüche 1 bis 5, **dadurch gekennzeichnet, dass** die Stoffmischung Cholin in einer Menge von 45 bis 55 mg umfasst.

8. Stoffmischung gemäß einem der Ansprüche 1 bis 7, **dadurch gekennzeichnet, dass** die Stoffmischung Ginseng in einer Menge von 40 bis 105 mg umfasst.

9. Stoffmischung gemäß einem der Ansprüche 1 bis 7, **dadurch gekennzeichnet, dass** die Stoffmischung Ginseng in einer Menge von 40 bis 60 mg umfasst.

10. Stoffmischung gemäß einem der Ansprüche 1 bis 9, **dadurch gekennzeichnet, dass** die Stoffmischung Pantothensäure in einer Menge von 2 bis 7 mg umfasst.

11. Stoffmischung gemäß Anspruch 3, **dadurch gekennzeichnet, dass** die Stoffmischung DMAE in einer Menge von 45 bis 55 mg, Cholin in einer Menge von 45 bis 55 mg und Ginseng in einer Menge von 40 bis 60 mg umfasst.

12. Stoffmischung gemäß Anspruch 3, **dadurch gekennzeichnet, dass** die Stoffmischung Pyridoxin in einer Menge von 0,2 bis 2,1 mg, DMAE in einer Menge von 45 bis 55 mg, Cholin in einer Menge von 45 bis 55 mg und Ginseng in einer Menge von 40 bis 60 mg umfasst.

13. Stoffmischung gemäß Anspruch 3, **dadurch gekennzeichnet, dass** die Stoffmischung Thiamin in einer Menge von 0,4 bis 1,31 mg, DMAE in einer Menge von 45 bis 55 mg, Cholin in einer Menge von 45 bis 55 mg, Pantothensäure in einer Menge von 2 bis 7 mg, Riboflavin in einer Menge von 0,3 bis 2 mg und Pyridoxin in einer Menge von 0,2 bis 2,1 mg umfasst.

14. Stoffmischung gemäß Anspruch 3, **dadurch gekennzeichnet, dass** die Stoffmischung Magnesiumoxid in einer Menge von 140 mg bis 160 mg, DMAE in einer Menge von 45 bis 55 mg, Cholin in einer Menge von 45 bis 55 mg, Pantothensäure in einer Menge von 2 bis 7 mg, Riboflavin in einer Menge von 0,3 bis 2 mg, Pyridoxin in einer Menge von 0,2 bis 2,1 mg und Thiamin in einer Menge von 0,4 bis 1,31 mg umfasst.

15. Verwendung der Stoffmischung gemäß einem der Ansprüche 1 bis 14 als Nahrungsergänzungsmittel.
